# EUROPEAN PATENT APPLICATION

(11) **EP 0 909 755 A1**
(43) Date of publication of application: **21.04.1999**
(21) Application number: 98117609.2
(22) Date of filing: 17.09.1998
(51) Int. Cl.: C07C 323/52, C10M 135/26

(54) **Thio-/mercapto-derivatives and use as antioxidant additives**

(30) Priority: 26.09.1997 US 938650
(71) Applicant: EXXON RESEARCH AND ENGINEERING COMPANY, Florham Park, New Jersey 07932-0390 (US)
(72) Inventor: Francisco, Manuel A., Washington, NJ 07882 (US); Puckace, James S., Perrineville, NJ 08535 (US); Cameron, Stephen D., Milford, NJ 08848 (US); Polizzotti, Richard Samuel, Milford, NJ 08848-2153 (US)
(74) Representative: Somers, Harold Arnold

(57) **Abstract**

Defined oil-soluble said derivatives, more especially certain derivatives of pentaerithrytol, are described. They have active C-S bonds (those containing sulfur bonded to carbon having a tertiary hydrogen and an electron withdrawing group). Said derivatives may be used to enhance the oxidation-resistance of lubricating oils, fuels and greases.

## Description

The present invention relates to certain (sulfur) mercapto-/thio-derivatives, and to their use as antioxidants; more especially as antioxidants for lube oils and fuels, for example those employed in internal combustion engines, and for greases.

Antioxidants are added to lube oils to neutralize or minimize oil degradation chemistry. For example, U.S. Patent 5,200,101 discloses certain amine/hindered phenol, acid anhydride and thiol ester-derived products are multi-functional antioxidant, antiwear and rust inhibiting lube additives. However, there is a continuing need for new antioxidant additives. The present invention addresses these needs.

The present invention provides for formulated internal combustion engine lube oil compositions, comprising a major amount of a lubricating oil and a minor amount of an oil soluble antioxidant of the formula below. The minor amount should be effective to enhance the antioxidancy of the lubricating oil.

The compounds of the present invention are a group of compounds that contain a sufficient sulfur density (per mole or weight percent), labile S―C bonds and oil compatibility (e.g., solubility, dispersibility).

The compositions comprise compounds of the formula: 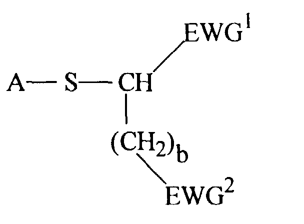 wherein EWG¹ and EWG² each is an independently selected electron withdrawing group and b is 0 (i.e., (CH₂)_{b} is absent) or 1. Preferably EWG¹ and EWG² each is an independently selected ―COOR group, however, suitable other EWG include, nitro nitrile, ketone, imide, SOₙR wherein n = 2 or 3, and amide groups. Others are known to those skilled in the art, for example, as disclosed in March, Advanced Organic Chemistry, 2nd ed. McGraw Hill. The resulting composition is represented by the formula: 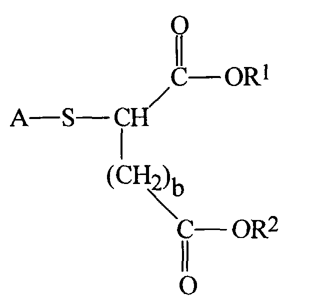 wherein A is selected from the group consisting of substituted and unsubstituted, branched or unbranched alkanes, alkynes, alkenes, aromatics and mixtures thereof and wherein b is 0 (i.e., (CH₂)_{b} is absent) or 1 and wherein R¹ and R² each is independently selected from H and C₁ to C₃₆ hydrocarbyl groups. Preferably, when A is an alkane it is a CH₃CH₂CH₂― group, preferably R¹ and R² are independently selected from CH₃. When a composition of the present invention is produced as a product of a specified reaction, the products occur as a distribution of reaction products. The formulas herein include isomers thereof. Typically, A is a substituted group having the formula 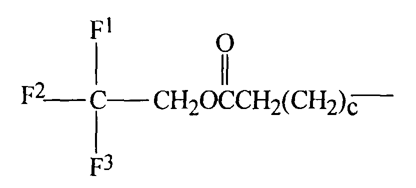 wherein F¹, F² and F³ are independently selected from the group consisting of 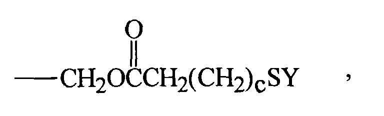 hydrocarbyl, H and OH groups, and mixtures thereof, and wherein c is 0 (i.e., ―(CH₂)_{b} is absent), 1, or 2;
and wherein when F¹, F² or F³ is 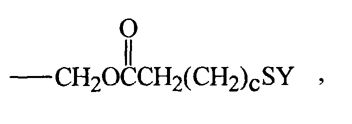 Y is independently selected from the group consisting of H and 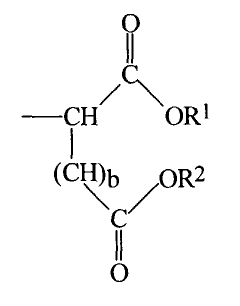 and wherein R¹ and R² each is independently selected from H and C₁ to C₃₆ hydrocarbyl groups, b is zero (i.e.,―(CH)_{b} is absent) or 1.

The present invention also provides for a method for enhancing the antioxidant properties of a lubricating oil which comprises adding to the lubricating oil a composition containing at least one compound of the above formulas.

The present invention also relates to the novel antioxidants of the above-referenced formulas and to a method of enhancing the antioxidancy of a lubricating oil by combining a major amount of a lubricating oil and a minor, effective amount of an oil soluble antioxidant of the above-referenced formulas to enhance the antioxidancy of the lubricating oil. Typically an amount of from about 0.05 to about 20 wt% of the additive is present, preferably from about 0.1 to about 15 wt%, in the formulated oil.

The present invention may suitably comprise, consist or consist essentially of the elements disclosed herein, and may be practiced in the absence of an element not specifically recited.

### DETAILED DESCRIPTION OF THE INVENTION

One embodiment of the present invention provides for a method of imparting enhanced antioxidancy to lubricating oils by combining a base or formulated oil of lubricating viscosity and an oil compatilizable (e.g., soluble or dispersible) antioxidant of the formula below in an amount sufficient or effective to enhance the antioxidancy of the base or formulated lube oil. These molecules are distinguished by the combination of oil compatilizable (e.g., soluble or dispersible); the presence of certain labile S―C bonds, i.e., in which the sulfur group is bonded to a carbon atom which is bonded to a tertiary hydrogen and to an electron withdrawing group; and a sufficient sulfur density (of labile S―C bond sulfur) to produce enhanced antioxidant activity. Thus in the present invention it has been discovered that compounds containing these certain labile S―C bonds provide enhanced antioxidancy in comparison to compounds (such as those disclosed in U.S. Patent No. 5,200,100) that do not contain such bonds.

A further embodiment of the present invention provides for formulated oil compositions containing a lubricating oil, and an antioxidancy enhancing amount of compounds having the formula specified below. A further embodiment of the present invention provides for novel compositions having enhanced antioxidancy of the formula specified below.

Included in the composition are isomers thereof. Additionally when the compositions are produced as the product of a specified reaction the products occur as a distribution of these reaction products.

More specifically, the present invention provides for:

A composition represented by the formula: 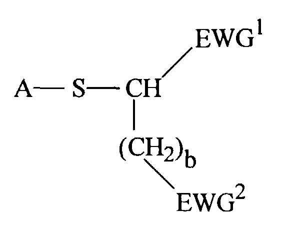 wherein EWG¹ and EWG² each is an independently selected electron withdrawing group and b is 0 (i.e., (CH₂)_{b} is absent) or 1.

Preferably EWG¹ and EWG² each is independently selected from ―COOR groups, however, suitable other EWG include nitro, nitrile, ketone, imide, SOₙR wherein n = 2 or 3 and amide groups. Preferably, the resulting composition is represented by the formula: 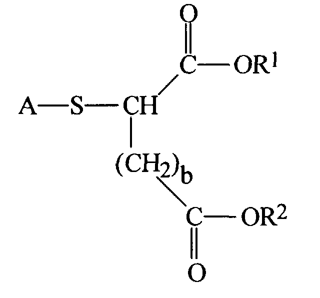 wherein A is selected from the group consisting of substituted or unsubstituted, branched or unbranched alkanes, alkynes, alkenes, aromatics and mixtures thereof and wherein b is 0 (i.e., (CH₂)_{b} is absent) or 1 and wherein R¹ and R² each is independently selected from H or C₁ to C₃₆ hydrocarbyl groups. Preferably, when A is an alkane it is a CH₃CH₂CH₂― group, preferably R¹ is H, b is 1, and R² is CH₃.

Typically, A is a substituted group having the formula 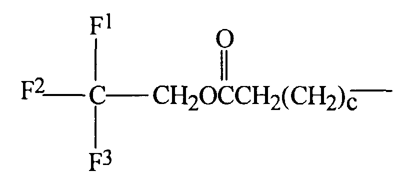 wherein F¹, F² and F³ are independently selected from the group consisting of 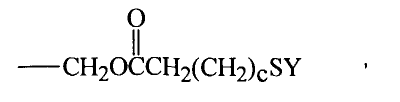 hydrocarbyl, H, and OH groups, and mixtures thereof, and wherein c is 0 (i.e., (CH₂)_{c} is absent), 1, or 2;
and wherein when F is 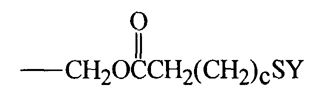 Y is independently selected from the group consisting of H and 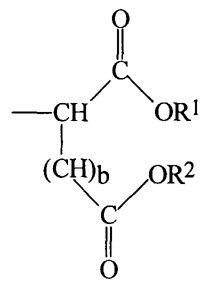 and wherein R¹ and R² each is independently selected from H and C₁ to C₃₆ hydrocarbyl groups, and b is zero (i.e., (CH)_{b} is absent) or 1. Preferably R¹ and R² are the same and preferably b is 1.

The present invention also provides for a method for enhancing the antioxidant properties of a lubricating oil which comprises adding to the lubricating oil a composition containing a sulfur dense molecule wherein said sulfur is bonded to a carbon having a tertiary hydrogen bond, and wherein said carbon also is bonded to an electron withdrawing group.

The organic groups (R¹, R² and (CH)_{b}) in the molecule should be of sufficient length to impart oil solubility or dispersibility to the molecule, particularly in view of the nature and type of sulfur functionalities and electron withdrawing groups present, Most desirably the compounds are or can be rendered oil soluble or dispersible at the conditions in which they are used.

In the present invention preferred antioxidant compounds derived from pentaerythritol and maleic anhydride of the above formula are exemplified by the following compounds:

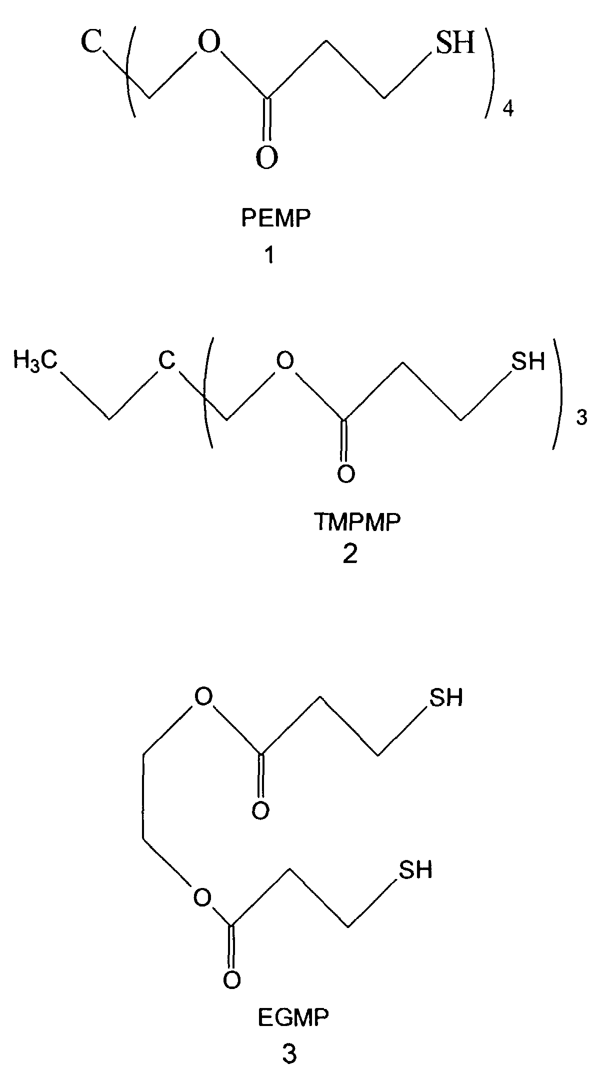

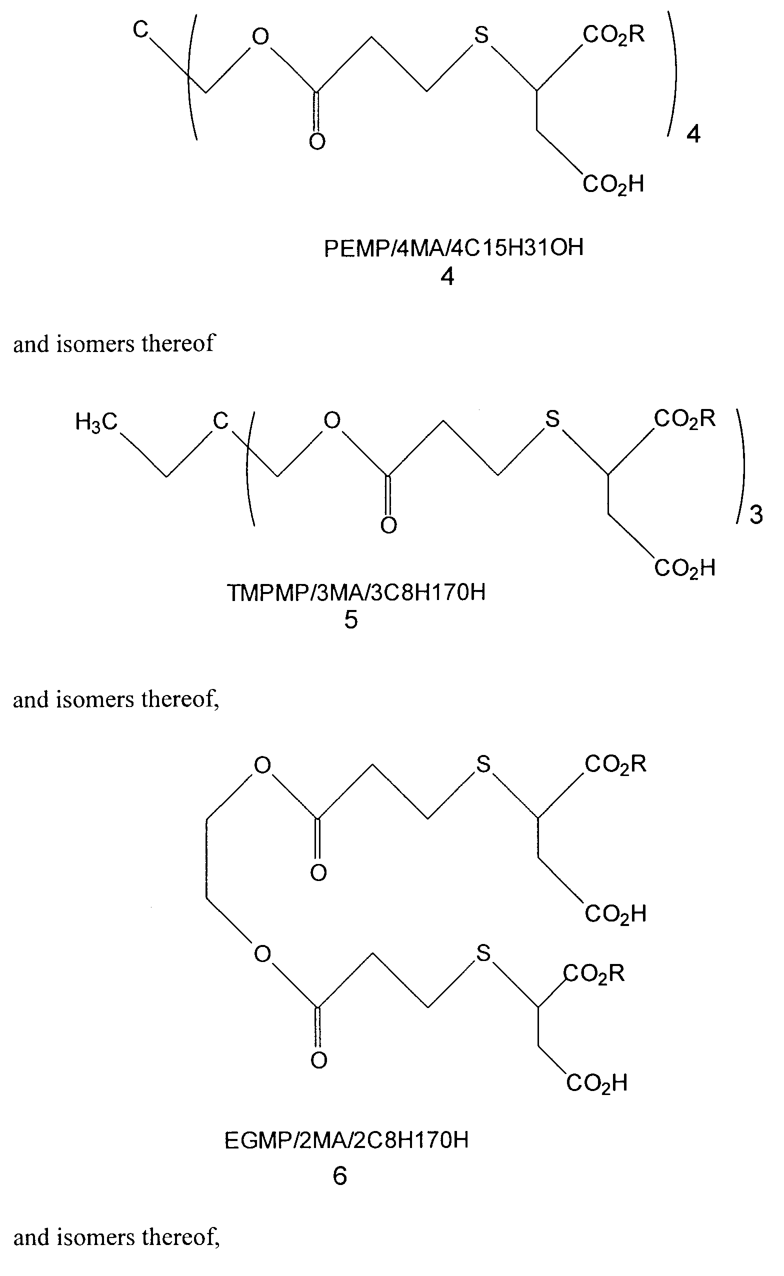

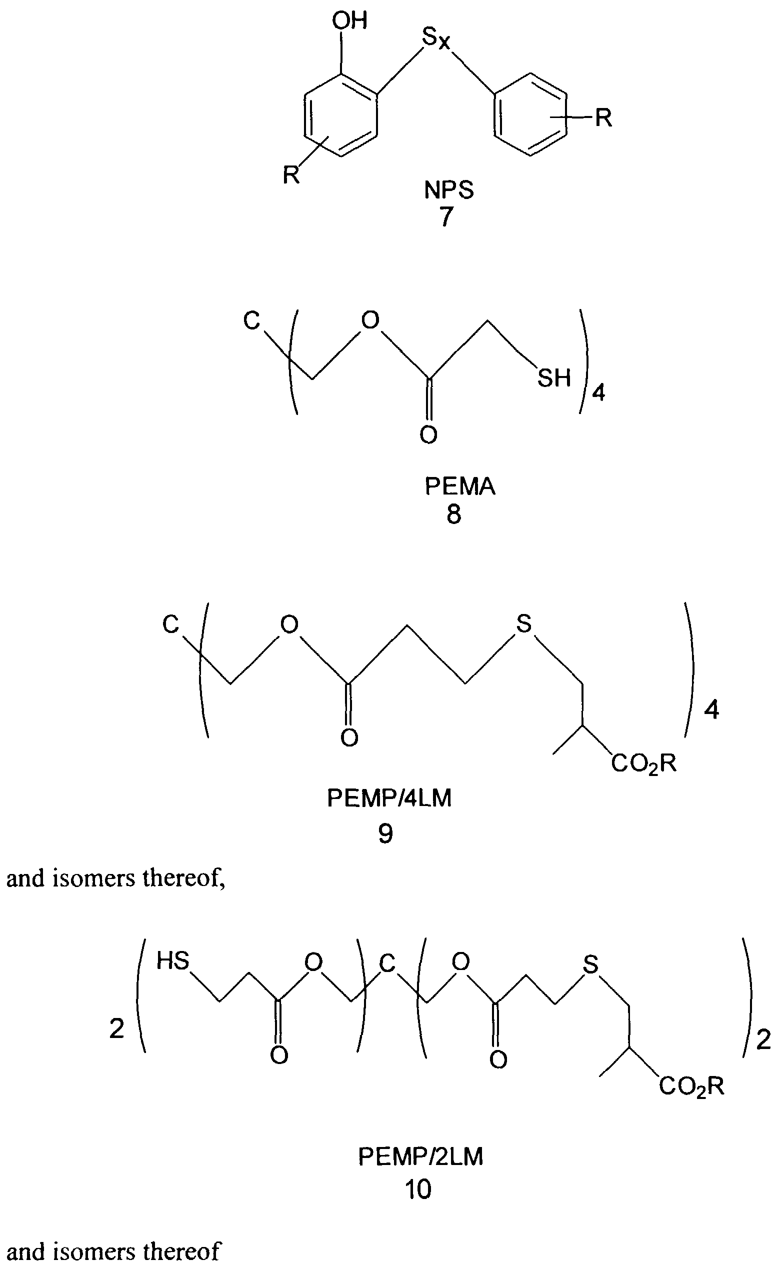

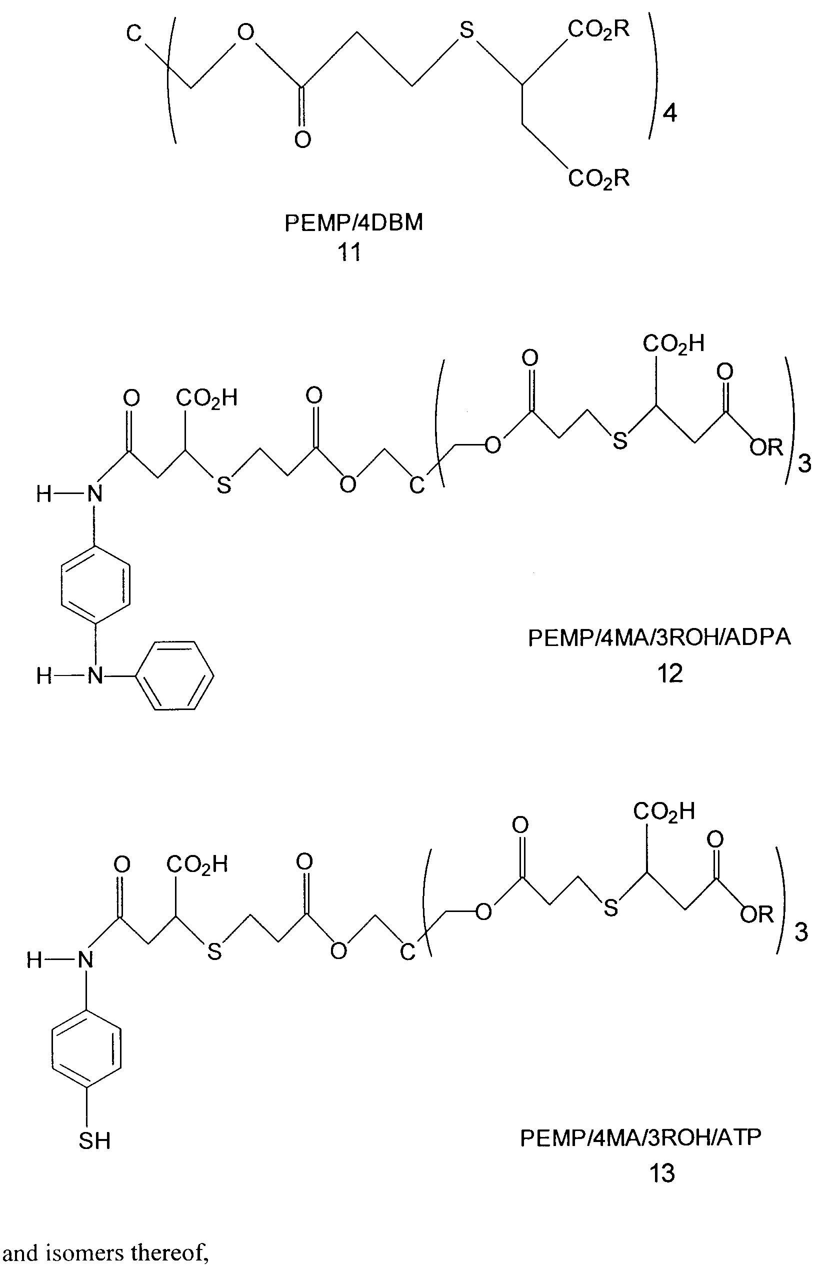

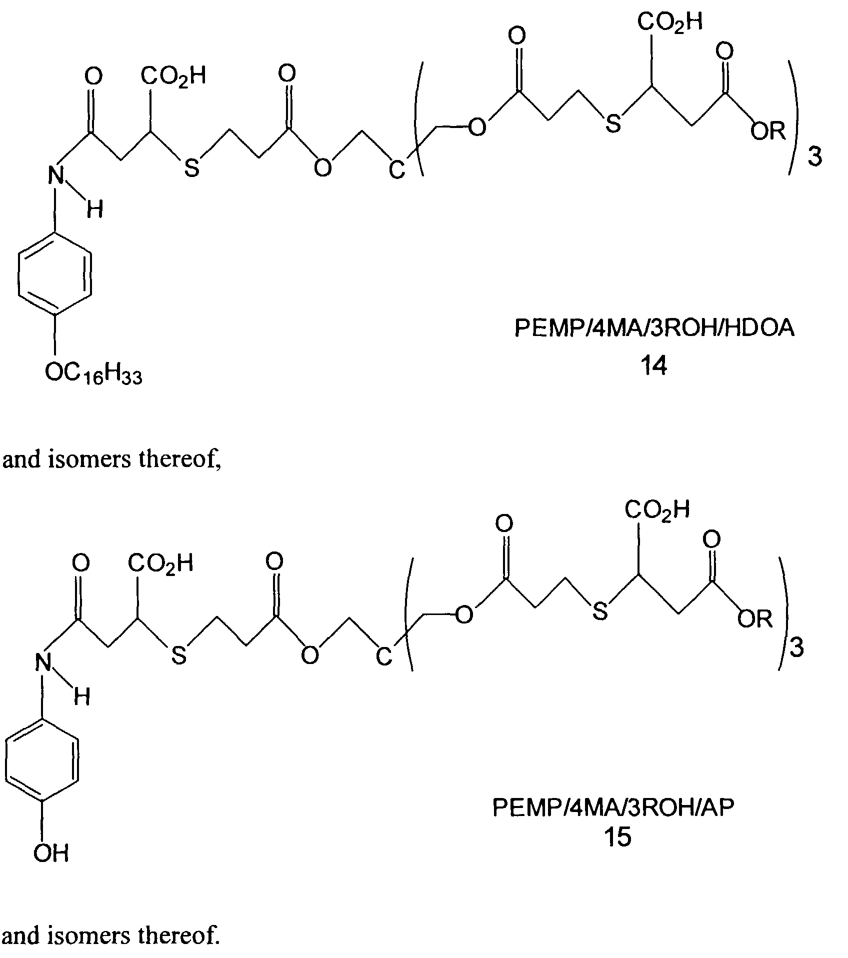

As used in the preceding formulas 1-15, R is any suitable hydrocarbyl or organic group referred to in the preceding formulas as being R¹ or R².

Examples include C₁₇H₂₈O₈S₄ (pentaerythritol tetrakis (3-mercaptopropionate)) and the ester-derivatized thiols and ester hydrolyzed monocarboxylic acid thiol derivatives of pentaerythritol tetrakis (3-mercaptopropionate), particularly those represented by the formulas C₃₇H₆₄O₁₂S₅ and C₆₁H₁₀₈O₂₄S₄. More particularly C₃₇H₆₄O₁₂S₅ may be exemplified by the formula below and includes isomers thereof: 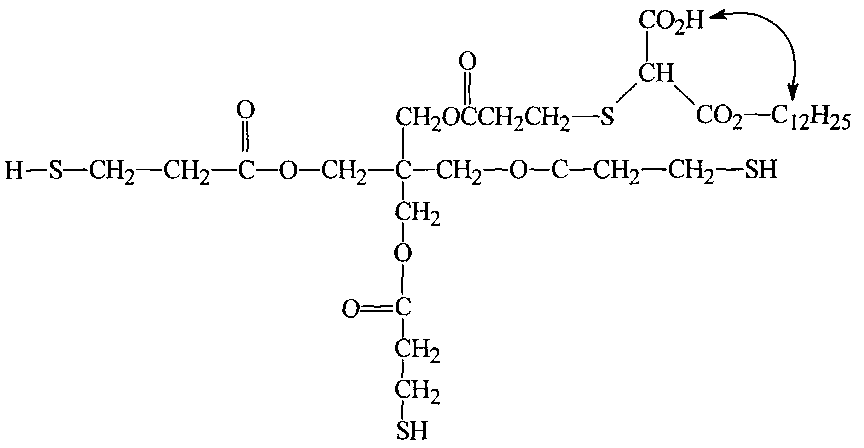 and C₆₁H₁₀₈O₂₄S₄ may be exemplified by the formula below and includes isomers thereof:

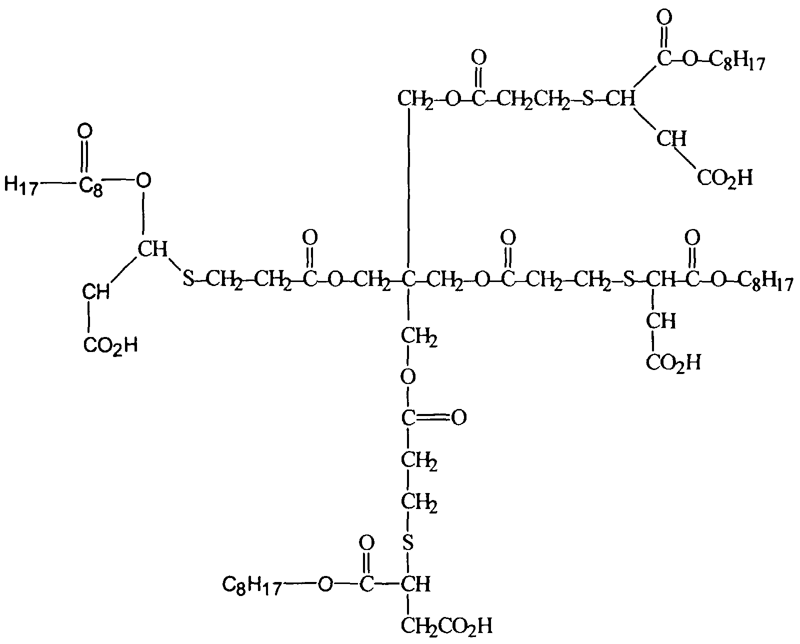

Additional preferred compounds include:

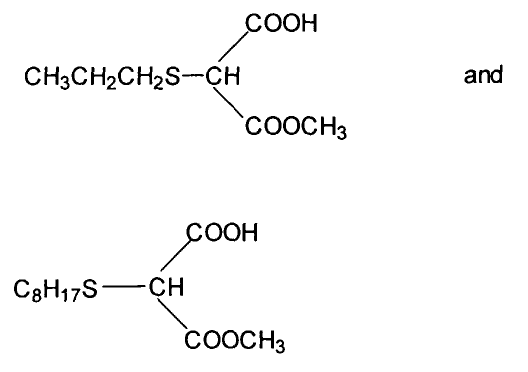

Generally the compounds of the present invention may be prepared by a process that produces a reaction product of an alkanethiol, maleic anhydride, and an alcohol. A reactor is charged with the maleic anhydride and the corresponding alcohol source and is heated above the melt temperature of maleic anhydride under a nitrogen blanket. The thiol source and a catalytic amount of a non-nucleophilic base (such as triethylamine) are introduced. The reaction is carried out for an appropriate length of time and is completed by adding a catalytic amount of a free radical initiator (such as azo bis-isobutyl nitrile), and heated at the reflux temperature of the alcohol up to 150°C for an appropriate time to produce the reaction product. The process may be adapted for preparing maleate analogs. The process typically yields an isomeric mixture of the reaction products. Typically these can be obtained in over 90% yield.

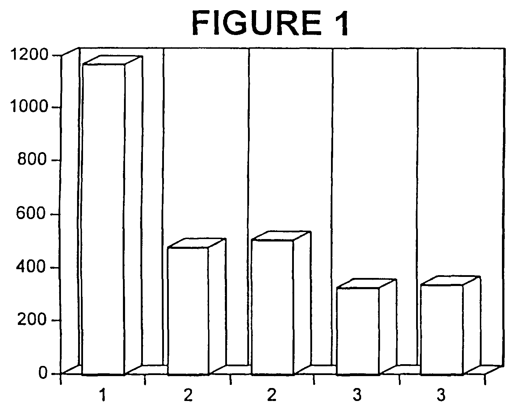

The additives have the ability to enhance the oxidation resistance (antioxidancy) characteristics of various oleagenous materials used in automotive and similar applications, particularly lube oils.
The Figures illustrate the essential features of the additives of the present invention. The numbers under each column in each Figure indicate the compound used and correspond to the parenthetically numbered chemical formulas in the text above. Figure 1 is a plot of moles of cumene hydroperoxide (CHP) decomposed (a measure of antioxidant performance) per mole of additive (y-axis) for various additives in S150/S100N oil with CHP at 125°C (x-axis) and indicates that greater sulfur density gives higher antioxidant activity. Thus PEMP is a more effective antioxidant than TMPMP or EGMP.

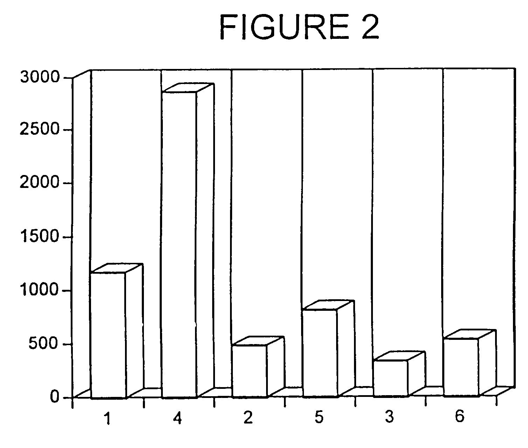

Figure 2 is a plot of moles of CHP decomposed per mole of additive (y-axis) for various additives in S150N/S100N with CHP at 125°C (x-axis) and shows that additives with increased oil compatibilizbility due to the presence of R groups that provide oil soluble and function give higher antioxidant activity. This PEMP derivatized with MA and C₁₅H₃₁OH is more effective than PEMP, TMPMP and EGMP and the MA and longer chain organic alcohols are more effective than the underivatized molecule.

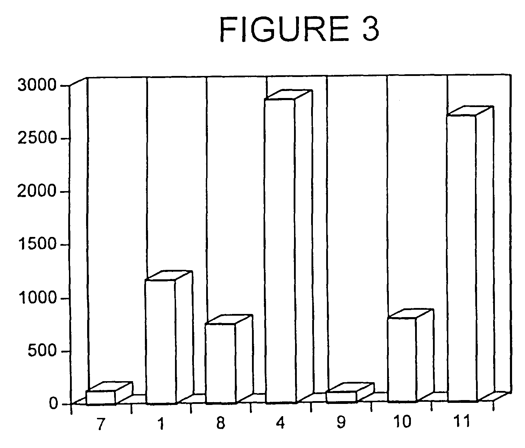

Figure 3 is a plot of moles of CHP decomposed per mole of additive (y-axis) for various additives in S150N/S100 with CHP at 125°C (x-axis) and shows that additives with labile S-C bonds give higher antioxidant activity. Thus, the sulfurized antioxidant, NPS, which contains aromatic sulfur and is one typically-used lube additive that does not contain the required labile S-C bond(s). As the number of labile S-C bonds increases antioxidant performance also increased (compare reaction products of PEMP vs. TMPMP vs. EGMP with the typically-used antioxidant, NPS).

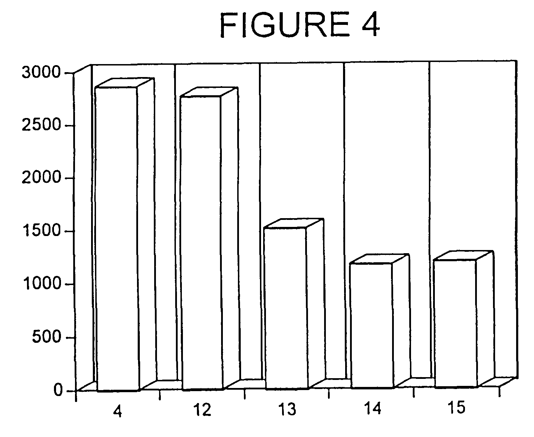

Figure 4 is a plot of moles of CHP decomposed per mole of additive (y-axis) for various additives in S150N/S100N with CHP at 125°C (x-axis) and shows that additives not possessing the sulfur density, S-C bond liability and oil solubility of the additives of the present invention show lower antioxidant activity, i.e., incorporation of antioxidant functionality (moiety) such as DPA, ATP, HDOA and AP provide lower antioxidant performance than PEMP/MA/C₁₅H₃₁OH.

Suitable oleagenous materials include hydrocarbyl lubricating media which may comprise liquid oils in the form of either a mineral or synthetic oil, or in the form of a grease in which the aforementioned oils are employed as a vehicle.

Generally, the formulated oil compositions used in accordance with the invention comprise a major amount of a base or formulated oil of lubricating viscosity and a minor amount of the additive. The term "minor amount" means an amount of less than 50% by weight of the composition. The term "major amount" means an amount of more than 50% by weight of the composition. More particularly, the additives of the invention are present in an amount that is sufficient to retard oxidation of the hydrocarbon (i.e., base or formulated oil) to which it is added and may also impart other properties as noted below. Typically, the minor amount of additive in proportion to the base or formulated oil will be effective in order to impart antioxidancy properties and ranges from about 0.05% to about 20% by weight, preferably 0.1% to 15% by weight of the composition.

The additives of the present invention may be added to produce the formulated oils of the present invention by any of the methods known to the art.

The lubricant oil is typically utilized at about 75% to about 99.5% by weight of the composition, preferably about 80% to about 99% by weight. Diluent oils present as various additives are included in the above amounts. Where the lubricant is to be used in the form of a grease the lubricating oil is generally employed in an amount sufficient to balance the total grease composition after accounting for the desired quantity of thickening agent and other additive components to be used in the grease. Suitable thickening agents are those known in the art.

The oils of lubricating viscosity utilized in the preparation of the lubricants for use in the invention may be based on natural oils, synthetic oils, or mixtures thereof. Natural oils include animal oils and vegetable oils as well as mineral lubricating oils such as liquid petroleum oils and solvent-treated or acid-treated mineral lubricating oils of the paraffinic, naphthenic or mixed paraffinic-naphthenic types. Oils of lubricating viscosity derived from coal or shale are also useful. Synthetic lubricating oils include hydrocarbon oils and halo-substituted hydrocarbon oils such as polymerized and interpolymerized olefins poly(1-hexenes), poly(1-octenes), poly(1-decenes), etc. and mixtures thereof; alkylbenzenes; polyphenyls alkylated diphenyl ethers and alkylated diphenyl sulfides and the derivatives, analogs and homologs thereof and the like. Unrefined, refined and rerefined oils, either natural or synthetic (as well as mixtures of two or more of any of these) of the type disclosed hereinabove can be used in the present invention. Unrefined oils are those obtained directly from a natural or synthetic source without further purification treatment. For example, a shale oil obtained directly from retorting operations, a petroleum oil obtained directly from primary distillation or ester oil obtained directly from an esterification process and used without further treatment would be an unrefined oil. Refined oils are similar to the unrefined oils except they have been further treated in one or more purification steps known in the art to improve one or more properties. Rerefined (i.e., reclaimed or reprocessed) oils are obtained by processes similar to those used to obtain refined oils applied to refined oils, but often are additionally processed by techniques directed to removal or spent additives and oil breakdown products. Most preferably, the oil used herein is a petroleum derived oil.

When used as an antioxidant in fuels, the concentration range may be any amount within the range typically used for antioxidants. However, practically the upper limit will be constrained by regulations related to sulfur content in fuels.

Other additives typically added to lubricating oils also may be present. Such conventional additive types include viscosity modifiers, extreme pressure agents, corrosion-inhibiting agents, pour point depressants, detergents, dispersants, color stabilizing agents, anti-foam agents, and other such additive materials known generally to those skilled in the art of formulating diesel lubricants.

Herein the convention used for designating reaction products is as follows: compound/derivatizing agent/derivatizing agent/derivatizing agent. Thus by way of example: PEMP/MA/C₁₅H₃₁OH indicates PEMP dervatized with 1 equivalent of maleic anhydride and 1 equivalent of C₁₅H₃₁OH; PEMP/4MA/3ROH/ATP means PEMP derivatized with 4 equivalents of maleic anhydride, 3 equivalents of alcohol and one equivalent of ATP.

The following abbreviations are used herein:

| Abbreviation | Meaning |
|---|---|
| PEMP | Pentaerythritol tetrakis (3-mercaptopropionate) |
| MA | Maleic anhydride |
| LM | Lauryl maleate |
| ADPA | Amino diphenyl amine |
| DPA | Diphenyl amine |
| ATP | Aminothiophenol |
| HDOA | Hexadecyloxyaniline |
| DBM | dibutyl maleate |
| PEMA | pentaerythritol mercaptoacetate |
| TMPMP | trimethylol propane mercaptoproprionate |
| EGMP | ethylene glycol mercaptopropionate |
| EGMA | ethylene glycol mercaptoacetate |
| AP | Aminophenol |
| NPS | nonylphenyl sulfide |

The present invention is exemplified by reference to the following examples:

### Example 1: Preparation of C₃₇H₅₄O₁₁S₅ derivative of pentaerythritol tetrakis (3-mercaptopropionate).

Solutions of the pentaerythritol-tetrakis (3-mercaptopropionate), C₁₇H₂₈O₈S₄, and acetylenedicarboxylic acid (ADCA) were dissolved in dioxane at room temperature to a 1 C₁₇H₂₈C₈S₄:4 (ADCA) molar ratio and mixed together followed by the addition of 0.02 mole % of the catalyst triethylamine (TEA). The mixture was heated to 60°C for one hour with stirring then allowed to cool to room temperature to form C₃₃H₃₆O₂₄S₄. After 16 hours an amount of dodecyl mercaptan (C₁₂H₂₅SH) equal to the number of moles of the ADCA was added to the solution to add to the remaining double bonds yielding the C₈₁H₁₄₀O₂₄S₈ derivative of PEMP, which was isolated by vacuum distillation at 70°C to remove excess solvent.

### Example 2: Preparation of C₆₅H₁₀₈O₂₄S₄ derivative of pentaerythritol tetrakis (3-mercaptopropionate)

Solutions of PEMP, C₁₇H₂₈O₈S₄, and maleic anhydride (MA) were dissolved in minimum volumes of tetrahydrofuran (THF) at room temperature to a 1 C₁₇H₂₈O₈S₄:4MA molar ratio. These solutions were mixed together followed by the addition of 0.02 mole% of the catalyst triethylamine (TEA), heated to 60°C for one hour with stirring, then allowed to cool to room temperature. After 16 hours an amount of n-octanol, C₈H₁₇OH, equal to the number of moles of the MA was added to the solution to open the anhydride and form, C₆₅H₁₀₈O₂₄S₄, the n-octyl hemiester derivative in the Formula (2) above, which was isolated by vacuum distillation at 70°C to remove excess solvent.

### Example 3: Preparation of 2-(thiopropyl)-1,4-butanedioic acid, 1-methylester and 2-(thiopropyl)-1,4-butanedioic acid, 4-methylester mixture

A four necked flask equipped with a thermometer, an addition funnel, a stirrer and a reflux condenser was flamed and swept with nitrogen. Maleic anhydride (49 g; 0.5 mole) was charged. Methanol (21 g; 0.654 mole) was then added and the reactor heated to reflux. In the addition funnel, n-propyl mercaptan (61 g; 0.8 mol) and triethyl amine (1 g; 7 mmole) were mixed. The mixture is slowly added. The resultant mixture was refluxed for 6 hours. Vaso-64 (0.025 g; 0.15 mmole), a commercial free radical initiator was added to the reactor and the mixture was soaked at reflux for an additional 3 hours. 13C NMR Spectroscopy of the reactor contents reflected a 90% yield of the desired mixture of isomers.

### Example 4: Preparation of 2-(thiopropyl)-1,4-butanedioic acid, 1,4-methyldiester

A four-necked flask equipped with a thermometer, an addition funnel, a stirrer and a reflux condenser was flamed and swept with nitrogen. Dibutyl maleate (114 g; 0.5 mole) was charged. In the addition funnel n-propyl mercaptan (61 g; 0.8 mol) and triethyl amine (1 g; 7 mmole) were mixed. The mixture was slowly added. It is then heated to 95°C for 6 hours. Vaso-66 (0.025 g; 0.15 mmole), a commercial free radical initiator, was added to the reactor and the mixture was soaked at reflux for an additional 3 hours.

## Claims

1. A composition of the formula: 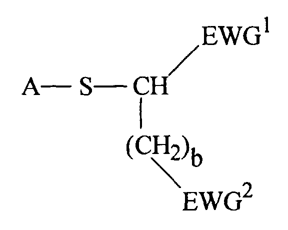 wherein EWG¹ and EWG² each is an independently selected electron-withdrawing group, b is 0 or 1, and A is selected from substituted and unsubstituted alkanes, alkenes, alkynes, aromatics and mixtures thereof.

2. The composition of claim 1, wherein EWG¹ and EWG² each is an independently selected ―COOR group, wherein the resulting composition has the formula 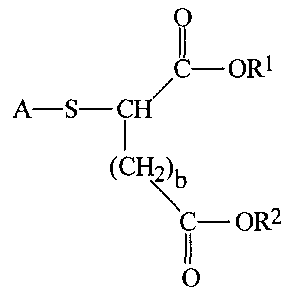 wherein A is selected from substituted and unsubstituted, branched or unbranched alkanes, alkynes, alkenes, aromatics and mixtures thereof and wherein b is 0 or 1 and wherein R¹ and R² each is independently selected from H and C₁ to C₃₆ hydrocarbyl groups.

3. The composition of claim 1 or claim 2, wherein A is an alkane.

4. The composition of claim 2 or claim 3, wherein the alkane is a CH₃CH₂CH₂― group.

5. The composition of any one of claims 2 to 4, wherein R¹ and R² are independently selected from CH₃ and H.

6. The composition of any preceding claim, wherein b is 1.

7. The composition of any preceding claim, wherein A is a substituted group having the formula: 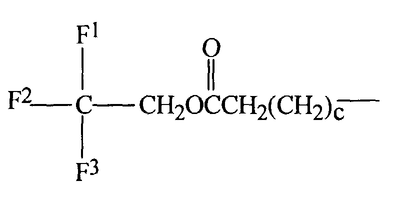 wherein F¹, F² and F³ are independently selected from 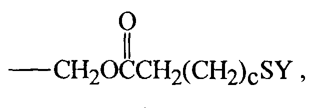 hydrocarbyl, H and OH groups, and mixtures thereof and wherein c is 0, 1, or 2, and
wherein when F¹,F² or F³ is 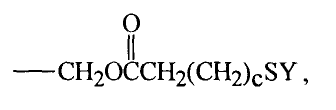 Y is independently selected from H and 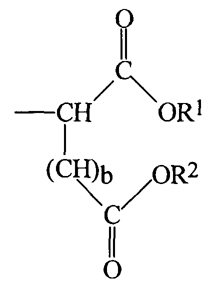 and wherein R¹ and R² each is independently selected from H and C₁ to C₃₆ hydrocarbyl groups, b is 0 or 1.

8. The composition of claim 7, wherein R¹ and R² are the same and b is 1.

9. The composition of claim 1, selected from structures represented by the formula and isomers thereof:

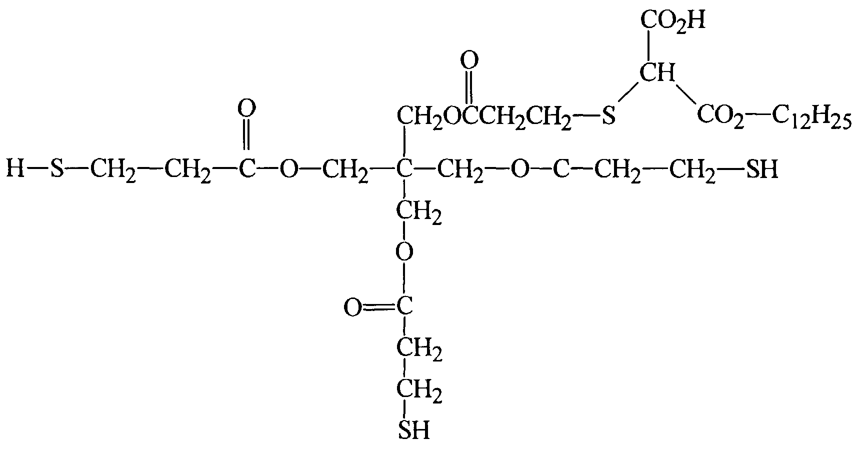

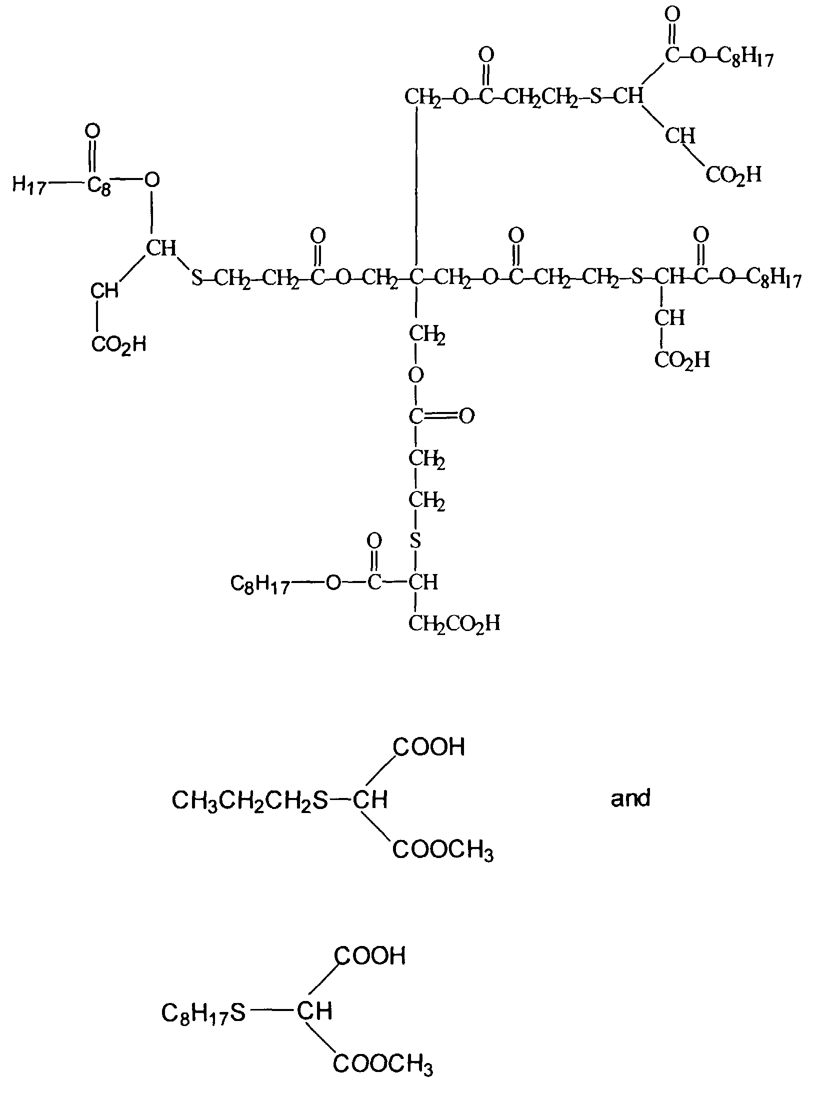

10. A lubricating oil, fuel or grease, composition comprising a major amount of lubricating oil, fuel or grease and a lesser amount of at least one of the compositions of any preceding claim.

11. A composition as claimed in claim 10, wherein said lesser amount is sufficient to impart antioxidant properties.

12. A lubricating oil according to claim 10 or claim 11, further comprising at least one conventional additive selected from detergents, dispersants, antiwear agents, antioxidants, corrosion inhibitors, antifoam agents and pour point depressants.

13. A lubricating oil according to any one of claims 10 to 12, wherein said lubricating oil contains less than 0.06 wt % phosphorous based on the weight of the finished oil.

14. A lubricating oil according to any one of claims 10 to 13, wherein said major amount of said lubricating oil is selected from hydrotreated oils and hydrocracked oils.

15. A method for improving the antioxidancy of a lubricating oil, fuel or grease, comprising adding thereto an effective amount of the composition of any one of claims 1 to 9.

16. A composition produced by the process of:
(a) heating an alkanethiol, maleic anhydride and an alcohol source at a temperature above the melt temperature of maleic anhydride;
(b) introducing a thiol source and a catalytic amount of a non-nucleophilic base;
(c) reacting the product of step (b) with a catalytic amount of a free radical initiator to produce a reaction product;
(d) stripping any excess thiol or alcohol from the product of step (c).
